# EUROPEAN PATENT APPLICATION

(11) **EP 2 514 437 A1**
(43) Date of publication of application: **24.10.2012**
(21) Application number: 12165482.6
(22) Date of filing: 20.07.2007
(51) Int. Cl.: A61K 39/145, C12N 7/00

(54) **Frozen stockpiling of influenza vaccines**

(30) Priority: 20.07.2006 GB 0614460
(62) Divisional of application: 07825298.8
(71) Applicant: Novartis AG, 4056 Basel (CH)
(72) Inventor: Contorini, Mario, I-53100 Siena (IT)
(74) Representative: Marshall, Cameron John

(57) **Abstract**

A problem with stockpiling influenza vaccines is their short shelf life. Storage volume is another problem. The invention prepares and stockpiles bulk vaccine, and the bulk is stored under frozen conditions. Compared to final vaccine, the higher antigen concentration and absence of packaging materials (boxes, vials, *etc*.) in the bulk means that the storage space requirements are hugely reduced. A process for preparing an influenza vaccine bulk thus comprises the steps of: (a) preparing bulk vaccine antigen from a source of influenza virus; and (b) storing the bulk vaccine prepared in step (a) under frozen conditions.

## Description

All documents cited herein are incorporated by reference in their entirety.

### TECHNICAL FIELD

This invention is in the field of manufacturing vaccines against influenza virus infection.

### BACKGROUND ART

Influenza vaccines currently in general use are described in more detail in chapters 17 & 18 of reference 1. They are based on live virus or inactivated virus, and inactivated vaccines can be based on whole virus, 'split' virus or on purified surface antigens (including haemagglutinin and neuraminidase). Haemagglutinin (HA) is the main immunogen in inactivated influenza vaccines, and vaccine doses are standardized by reference to HA levels, with vaccines typically containing about 15µg of HA per strain.

In a pandemic influenza outbreak then a large number of doses of influenza vaccine will be needed, but it will be difficult to increase vaccine supply to meet the huge demand. To avoid the need to prepare these vaccines once an outbreak has begun, it has therefore been proposed to prepare stockpiles of influenza vaccines in anticipation of a pandemic outbreak.

As noted in reference 2, however, one problem with such stockpiles is the short shelf-life of vaccines, despite the use of refrigeration. It is an object of the invention to provide ways in which the short shelf-life could be overcome when stockpiling influenza vaccines.

Vaccine stockpiles also create logistical problems. A typical vaccine box is about 80x40x40 mm, and so 10 million vaccine doses require about 1,250m³ (1,250,000 liters) of refrigerated storage space. It is a further object of the invention to provide ways in which these logistical problems can be avoided.

### DISCLOSURE OF THE INVENTION

The manufacturing process for current trivalent split or subunit influenza vaccines typically involves the following steps: growth of virus; purification of virus; inactivation of virus; disruption of virions or purification of surface glycoproteins to give bulk antigen; dilution of antigens to give final HA concentration of 15µg/dose; repetition of these steps for two other strains; combination of antigens from the three strains; dose filling; and packaging.

Rather than carrying out all of these steps, to permit stockpiling of finished vaccine product, the present invention prepares and stockpiles bulk vaccine. The later steps in vaccine manufacture can be carried out very quickly and so, in the event of a pandemic outbreak, the bulk could mobilized to allow rapid preparation of final vaccines while avoiding the time-consuming steps of virus growth, purification and inactivation. Moreover, the bulk vaccine can be stored under conditions that extend the shelf-life relative to final vaccines (*e.g.* it can be frozen), while the higher antigen concentration and absence of packaging materials (boxes, vials, *etc*.) means that the storage space requirements are hugely reduced *e.g.* the material for 10 million monovalent vaccines at a bulk concentration of 500µg/ml could be stored in much less than 1m³. The invention is particularly useful in stockpiling for potential pandemic situations, but can also be used in inter-pandemic periods.

Thus the invention provides a process for preparing an influenza vaccine bulk, comprising the steps of: (a) preparing bulk vaccine antigen from a source of influenza virus; and (b) storing under frozen conditions the bulk vaccine prepared in step (a). The process may include a previous step of growing influenza virus, and so step (a) will involve preparing bulk vaccine antigen from the viruses grown in that step.

The invention also provides a process for preparing an influenza vaccine from such a frozen bulk vaccine, comprising these steps and then the steps of: (c) thawing the frozen bulk vaccine; and (d) preparing a final vaccine for administration to patients from the bulk vaccine.

The invention also provides a process for preparing an influenza vaccine, comprising the steps of: (a) obtaining a bulk vaccine that has been stored under frozen conditions; and (b) preparing a final vaccine for administration to patients from the bulk vaccine. If the vaccine obtained in step (a) is still frozen then a thawing step will be included within or before step (b).

### The influenza virus

The invention is performed on influenza viruses that have been grown on a suitable substrate. Substrates currently in use for growing influenza viruses include eggs and cell culture. The current standard method for influenza virus growth uses specific pathogen-free (SPF) embryonated hen eggs, with virus being purified from the egg contents (allantoic fluid). More recently, however, viruses have been grown in animal cell culture and, for reasons of speed and patient allergies, this growth method is preferred. If egg-based viral growth is used then one or more amino acids may be introduced into the allantoic fluid of the egg together with the virus [47].

When cell culture is used, the viral growth substrate will typically be a cell line of mammalian origin. Suitable mammalian cells of origin include, but are not limited to, hamster, cattle, primate (including humans and monkeys) and dog cells. Various cell types may be used, such as kidney cells, fibroblasts, retinal cells, lung cells, *etc.* Examples of suitable hamster cells are the cell lines having the names BHK21 or HKCC. Suitable monkey cells are *e.g.* African green monkey cells, such as kidney cells as in the Vero cell line. Suitable dog cells are *e.g.* kidney cells, as in the MDCK cell line. Thus suitable cell lines include, but are not limited to: MDCK; CHO; 293T; BHK; Vero; MRC-5; PER.C6; WI-38; *etc.* Preferred mammalian cell lines for growing influenza viruses include: MDCK cells [3-6], derived from Madin Darby canine kidney; Vero cells [7-9], derived from African green monkey *(Cercopithecus aethiops*) kidney; or PER.C6 cells [10], derived from human embryonic retinoblasts. These cell lines are widely available *e.g.* from the American Type Cell Culture (ATCC) collection [11], from the Coriell Cell Repositories [12], or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. As a less-preferred alternative to mammalian cell lines, virus can be grown on avian cell lines [*e.g.* refs. 13-15], including cell lines derived from ducks (*e.g.* duck retina) or hens. Examples of avian cell lines include avian embryonic stem cells [13,16] and duck retina cells [14]. Suitable avian embryonic stem cells, include the EBx cell line derived from chicken embryonic stem cells, EB45, EB 14, and EB 14-074 [17]. Chicken embryo fibroblasts (CEF) may also be used.

The most preferred cell lines for growing influenza viruses are MDCK cell lines. The original MDCK cell line is available from the ATCC as CCL-34, but derivatives of this cell line may also be used. For instance, reference 3 discloses a MDCK cell line that was adapted for growth in suspension culture ('MDCK 33016', deposited as DSM ACC 2219). Similarly, reference 18 discloses a MDCK-derived cell line that grows in suspension in serum-free culture ('B-702', deposited as FERM BP-7449). Reference 19 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 20 discloses MDCK cell lines with high susceptibility to infection, including 'MDCK.5F1' cells (ATCC CRL-12042). Any of these MDCK cell lines can be used.

For growth on a cell line, such as on MDCK cells, virus may be grown on cells in suspension [3,21,22] or in adherent culture. One suitable MDCK cell line for suspension culture is MDCK 33016 (deposited as DSM ACC 2219). As an alternative, microcarrier culture can be used.

Cell lines supporting influenza virus replication are preferably grown in serum-free culture media and/or protein free media. A medium is referred to as a serum-free medium in the context of the present invention in which there are no additives from serum of human or animal origin. Protein-free is understood to mean cultures in which multiplication of the cells occurs with exclusion of proteins, growth factors, other protein additives and non-serum proteins, but can optionally include proteins such as trypsin or other proteases that may be necessary for viral growth. The cells growing in such cultures naturally contain proteins themselves.

Cell lines supporting influenza virus replication are preferably grown below 37°C [23] *e.g.* 30-36°C during viral replication.

Where virus is grown on a cell line then the growth culture, and also the viral inoculum used to start the culture, is preferably free from (*i.e.* will have been tested for and given a negative result for contamination by) herpes simplex virus, respiratory syncytial virus, parainfluenza virus 3, SARS coronavirus, adenovirus, rhinovirus, reoviruses, polyomaviruses, birnaviruses, circoviruses, and/or parvoviruses [24]. Absence of herpes simplex viruses is particularly preferred.

The method for propagating virus in cultured cells generally includes the steps of inoculating the cultured cells with the strain to be cultured, and then cultivating the infected cells for a desired time period for virus propagation, such as for example as determined by virus titer or antigen expression (*e.g.* between 24 and 168 hours after inoculation). The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture (*e.g.* monolayers) may be removed either by freeze-thawing or by enzymatic action to increase the viral content of the harvested culture supernatants. Harvested fluids may then be either inactivated (see below). Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Proteases (typically trypsin) are generally added during cell culture to allow viral release, and the proteases can be added at any suitable stage during the culture.

Influenza virus strains used in vaccines change from season to season. In the current inter-pandemic period, trivalent vaccines are typical, including two influenza A strains (H1N1 and H3N2) and one influenza B strain. The invention can be used with inter-pandemic strains of this type, but it is more useful with viruses from pandemic strains (*i.e.* strains to which the vaccine recipient and the general human population are immunologically naive), such as H2, H5, H7 or H9 subtype strains (in particular of influenza A virus), and influenza vaccines for pandemic strains may be monovalent or may, for instance, be based on a normal trivalent vaccine supplemented by a pandemic strain. Depending on the season and on the nature of the antigen included in the vaccine, therefore, the invention may protect against one or more of HA subtypes H1, H2, H3, H4, H5, H6, H7, H8, H9, H10, H 11, H12, H 13, H14, H15 or H16. The invention may protect against one or more of influenza A virus NA subtypes N1, N2, N3, N4, N5, N6, N7, N8 or N9.

As mentioned above, the invention is particularly useful for use with pandemic strains. The characteristics of an influenza strain that give it the potential to cause a pandemic outbreak are: (a) it contains a new hemagglutinin compared to the hemagglutinins in currently-circulating human strains, *i.e.* one that has not been evident in the human population for over a decade (*e.g.* H2), or has not previously been seen at all in the human population (*e.g.* H5, H6 or H9, that have generally been found only in bird populations), such that the human population will be immunologically naïve to the strain's hemagglutinin; (b) it is capable of being transmitted horizontally in the human population; and (c) it is pathogenic to humans. A virus with H5 haemagglutinin type is preferred for immunising against pandemic influenza, such as a H5N1 strain. Other possible strains include H5N3, H9N2, H2N2, H7N1 and H7N7, and any other emerging potentially pandemic strains. Within the H5 subtype, a virus may fall into HA clade 1, HA clade 1', HA clade 2 or HA clade 3 [25], with clades 1 and 3 being particularly relevant.

Influenza virus strains used with the invention may be resistant to antiviral therapy (*e.g.* resistant to oseltamivir [26] and/or zanamivir), including resistant pandemic strains [27].

Influenza virus strains used with the invention may be attenuated. They may be temperature-sensitive. They may be cold-adapted. These three features are particularly useful when using live virus as an antigen.

The influenza virus may be a reassortant strain, and may have been obtained by reverse genetics techniques. Reverse genetics techniques [*e.g.* 28-32] allow influenza viruses with desired genome segments to be prepared *in vitro* using plasmids. Typically, they involve expressing (a) DNA molecules that encode desired viral RNA molecules *e.g.* from poll promoters, and (b) DNA molecules that encode viral proteins *e.g.* from polII promoters, such that expression of both types of DNA in a cell leads to assembly of a complete intact infectious virion. The DNA preferably provides all of the viral RNA and proteins, but it is also possible to use a helper virus to provide some of the RNA and proteins. Plasmid-based methods using separate plasmids for producing each viral RNA are preferred [33-35], and these methods will also involve the use of plasmids to express all or some (*e.g.* just the PB1, PB2, PA and NP proteins) of the viral proteins, with up to 12 plasmids being used in some methods.

To reduce the number of plasmids needed, a recent approach [36] combines a plurality of RNA polymerase I transcription cassettes (for viral RNA synthesis) on the same plasmid (*e.g.* sequences encoding 1, 2, 3, 4, 5, 6, 7 or all 8 influenza A vRNA segments), and a plurality of protein-coding regions with RNA polymerase II promoters on another plasmid (*e.g.* sequences encoding 1, 2, 3, 4, 5, 6, 7 or all 8 influenza A mRNA transcripts). Preferred aspects of the reference 36 method involve: (a) PB1, PB2 and PA mRNA-encoding regions on a single plasmid; and (b) all 8 vRNA-encoding segments on a single plasmid. Including the NA and HA segments on one plasmid and the six other segments on another plasmid can also facilitate matters.

As an alternative to using poll promoters to encode the viral RNA segments, it is possible to use bacteriophage polymerase promoters [37]. For instance, promoters for the SP6, T3 or T7 polymerases can conveniently be used. Because of the species-specificity of poll promoters, bacteriophage polymerase promoters can be more convenient for many cell types (*e.g.* MDCK), although a cell must also be transfected with a plasmid encoding the exogenous polymerase enzyme.

In other techniques it is possible to use dual poll and polII promoters to simultaneously code for the viral RNAs and for expressible mRNAs from a single template [38,39].

Thus the virus, particularly an influenza A virus, may include one or more RNA segments from a A/PR/8/34 virus (typically 6 segments from A/PR/8/34, with the HA and N segments being from a vaccine strain, *i.e.* a 6:2 reassortant), particularly when viruses are grown in eggs. It may also include one or more RNA segments from a A/WSN/33 virus, or from any other virus strain useful for generating reassortant viruses for vaccine preparation. Typically, the invention protects against a strain that is capable of human-to-human transmission, and so the strain's genome will usually include at least one RNA segment that originated in a mammalian (*e.g*. in a human) influenza virus. It may include NS segment that originated in an avian influenza virus.

The HA encoded by the influenza virus may be a natural HA as found in a wild-type virus, or it may have been modified. For instance, it is known to modify HA to remove determinants (*e.g.* hyper-basic regions around the cleavage site between HA1 and HA2) that cause a virus to be highly pathogenic in avian species, as these determinants can otherwise prevent viral growth in eggs.

As an alternative to making influenza vaccines from material derived from influenza virions, it is known to express proteins in heterologous recombinant hosts. For example, HA can be expressed in an insect cell line using a baculovirus vector [40,41], as can neuraminidase [42]. Rather than involve the steps of growing influenza virus then preparing bulk vaccine antigen from these viruses, the invention may therefore involve a step of growing a recombinant host that expresses an influenza virus antigen, and preparing bulk vaccine from the recombinant host.

### The bulk vaccine

The bulk vaccine may include live virus or inactivated virus.

Live viruses are known to retain immunogenicity after frozen storage, as MedImmune's FLUMIST™ product (trivalent live virus) is stored in a freezer at or below -15°C prior to use. Where the bulk vaccine includes live virus, the bulk will be prepared by a process that comprises growing the virus on a suitable substrate and then purifying virions from virion-containing fluids. For example, the fluids may be clarified by centrifugation, and stabilized with buffer (*e.g.* containing sucrose, potassium phosphate, and monosodium glutamate).

Where the bulk includes inactivated viruses, various procedures may be used to inactivate viruses grown on a substrate. The procedure may involve a virion purification step followed by an inactivation step, or the inactivation can take place at the same time as purification. For example, a purification process may involve zonal centrifugation using a linear sucrose gradient solution that includes detergent for simultaneous inactivation (*e.g.* splitting) of virions.

Where a step of viral inactivation is performed, chemicals may be used. Chemical methods for inactivating a virus include treatment with an effective amount of one or more of the following agents: detergents, formaldehyde, β-propiolactone, or UV light. Additional chemical means for inactivation include treatment with methylene blue, psoralen, carboxyfullerene (C60) or a combination of any thereof. Other methods of viral inactivation are known in the art, such as for example binary ethylamine, acetyl ethyleneimine, or gamma irradiation.

Split virions are obtained by treating virions with detergents (*e.g.* ethyl ether, polysorbate 80, deoxycholate, tri-*N*-butyl phosphate, Triton X-100, Triton N101, cetyltrimethylammonium bromide, *etc*.) to produce subvirion preparations, including the 'Tween-ether' splitting process. Methods of splitting influenza viruses are well known in the art *e.g.* see refs. 43-48, *etc.* Splitting of the virus is typically carried out by disrupting or fragmenting whole virus, whether infectious or non-infectious with a disrupting concentration of a splitting agent. The disruption results in a full or partial solubilisation of the virus proteins, altering the integrity of the virus. Preferred splitting agents are non-ionic and ionic (*e.g.* cationic) surfactants *e.g.* alkylglycosides, alkylthioglycosides, acyl sugars, sulphobetaines, betains, polyoxyethylenealkylethers, N,N-dialkyl-Glucamides, Hecameg, alkylphenoxy-polyethoxyethanols, quaternary ammonium compounds, sarcosyl, CTABs (cetyl trimethyl ammonium bromides), tri-*N*-butyl phosphate, Cetavlon, myristyltrimethylammonium salts, lipofectin, lipofectamine, and DOT-MA, the octyl- or nonylphenoxy polyoxyethanols (*e.g.* the Triton surfactants, such as Triton X-100 or Triton N101), polyoxyethylene sorbitan esters (the Tween surfactants), polyoxyethylene ethers, polyoxyethlene esters, *etc.* One useful splitting procedure uses the consecutive effects of sodium deoxycholate and formaldehyde and, as mentioned above, splitting can take place during initial virion purification (*e.g.* in a sucrose density gradient solution). Split virions can usefully be resuspended in sodium phosphate-buffered isotonic sodium chloride solution.

Purified surface antigen vaccines comprise the influenza surface antigens haemagglutinin and, typically, also neuraminidase. Processes for purifying these proteins from virions after viral growth are well known in the art.

Another form of inactivated influenza antigen is the virosome. Virosomes can be prepared by solubilization of influenza virus with a detergent followed by removal of the nucleocapsid and reconstitution of the membrane containing the viral glycoproteins. An alternative method for preparing virosomes involves adding viral membraneglycoproteins to excess amounts of phospholipids, to give liposomes with viral proteins in their membrane. The invention can be used to store bulk virosomes.

Haemagglutinin (HA) is the main immunogen in inactivated influenza vaccines, and vaccine doses are standardised by reference to HA levels, typically as measured by a single radial immunodiffusion (SRID) assay. Final vaccines typically contain about 15µg HA per strain, although lower doses are also used *e.g.* for children, or in pandemic situations. Fractional HA doses such as ½ (*i.e.* 7.5µg per strain), ¼ and ⅛ have been used [49,50], as have higher doses (*e.g.* 3x or 9x [51,52]). In the present invention, the HA concentration in stored inactivated bulk material will be higher than the concentration in a final vaccine. The HA concentration in the stored bulk will typically have be in the range of 75-1000µg/ml. Within this range, the concentration will generally be at least 100µg/ml *e.g.* ≥150µg/ml, ≥200µg/ml, ≥250µg/ml, ≥300µg/ml, ≥350µg/ml, ≥400µg/ml, ≥450µg/ml, ≥500µg/ml, ≥550µg/ml, ≥600µg/ml, ≥650µg/ml, ≥700µg/ml, ≥750µg/ml, *etc.* The concentration will depend on factors such as the influenza strain, the growth substrate, *etc.* Higher HA concentrations mean that a lower storage volume is required for a fixed number of final doses.

In addition to containing HA, the bulk will usually contain also neuraminidase (NA). In addition, the bulk may include matrix protein, in order to benefit from additional T cell epitopes. It may include M1 and/or M2 matrix protein, and including detectable levels of M1 matrix protein (or fragments thereof) is preferred. Nucleoprotein may also be present.

For live vaccines, dosing is measured by median tissue culture infectious dose (TCID₅₀) rather than HA content, and a TCID₅₀ of between 10⁶ and 10⁸ (preferably between 10^{6.5}-10^{7.5}) per strain is typical for final vaccines. Stored bulk material will have a higher TCID₅₀ *e.g.* at least 10⁹ or 10¹⁰ per ml of bulk.

Bulk compositions of live or inactivated antigens may include detergent *e.g.* a polyoxyethylene sorbitan ester surfactant (known as 'Tweens'), an octoxynol (such as octoxynol-9 (Triton X-100) or t-octylphenoxypolyethoxyethanol), a cetyl trimethyl ammonium bromide ('CTAB'), or sodium deoxycholate, particularly for a split or surface antigen vaccine. The detergent may be present only at trace amounts.

Whereas current influenza vaccines are trivalent, bulk vaccines stored according to the invention will generally be monovalent (although they may later be used to prepare multivalent vaccines). Storage of monovalent material maintains post-storage flexibility *e.g.* in choosing which bulks to mix if a multivalent vaccine is desired, *etc.* While the stored bulk may include antigens from more than one (*e.g.* 2, 3, 4 or more) influenza virus strains, therefore, it is preferred that it should contain antigen from a single strain, which may be an influenza B virus or, more preferably, an influenza A virus. Storage of influenza B virus is advantageous as it rarely changes from season-to-season, and so stored material can be used for more than one season. Storage of influenza A virus is advantageous because of potential pandemic outbreaks.

Where virus has been grown on a mammalian cell line then the bulk vaccine will advantageously be free from egg proteins (*e.g.* ovalbumin and ovomucoid) and from chicken DNA, thereby reducing allergenicity in the final vaccine product.

Where virus has been grown on a cell line then it is standard practice to minimize the amount of residual cell line DNA in the final vaccine, in order to minimize any oncogenic activity of the DNA. Thus a bulk preferably contains less than 10ng (preferably less than 1ng, and more preferably less than 100pg) of residual host cell DNA per 15µg of HA, although trace amounts of host cell DNA may be present. In general, the host cell DNA that it is desirable to exclude from compositions of the invention is DNA that is longer than 100bp.

Measurement of residual host cell DNA is now a routine regulatory requirement for biologicals and is within the normal capabilities of the skilled person. The assay used to measure DNA will typically be a validated assay [53,54]. The performance characteristics of a validated assay can be described in mathematical and quantifiable terms, and its possible sources of error will have been identified. The assay will generally have been tested for characteristics such as accuracy, precision, specificity. Once an assay has been calibrated (*e.g.* against known standard quantities of host cell DNA) and tested then quantitative DNA measurements can be routinely performed. Three principle techniques for DNA quantification can be used: hybridization methods, such as Southern blots or slot blots [55]; immunoassay methods, such as the Threshold™ System [56]; and quantitative PCR [57]. These methods are all familiar to the skilled person, although the precise characteristics of each method may depend on the host cell in question *e.g.* the choice of probes for hybridization, the choice of primers and/or probes for amplification, *etc.* The Threshold™ system from *Molecular Devices* is a quantitative assay for picogram levels of total DNA, and has been used for monitoring levels of contaminating DNA in biopharmaceuticals [56]. A typical assay involves non-sequence-specific formation of a reaction complex between a biotinylated ssDNA binding protein, a urease-conjugated anti-ssDNA antibody, and DNA. All assay components are included in the complete Total DNA Assay Kit available from the manufacturer. Various commercial manufacturers offer quantitative PCR assays for detecting residual host cell DNA *e.g.* AppTec™ Laboratory Services, BioReliance™, Althea Technologies, *etc.* A comparison of a chemiluminescent hybridisation assay and the total DNA Threshold™ system for measuring host cell DNA contamination of a human viral vaccine can be found in reference 58.

Contaminating DNA can be removed during bulk preparation using standard purification procedures *e.g.* chromatography, *etc.* Removal of residual host cell DNA can be enhanced by nuclease treatment *e.g.* by using a DNase. A convenient method for reducing host cell DNA contamination during bulk manufacture is disclosed in references 59 & 60, involving a two-step treatment, first using a DNase (*e.g.* Benzonase), which may be used during viral growth, and then a cationic detergent (*e.g.* CTAB), which may be used during virion disruption.

Material containing <10ng (*e.g.* <1ng, <100pg) host cell DNA per 15µg of HA are preferred. Material containing <10ng (*e.g.* <1ng, <100pg) host cell DNA per 50µg of HA are more preferred. It is preferred that the average length of any residual host cell DNA is less than 500bp *e.g.* less than 400bp, less than 300bp, less than 200bp, less than 100bp, *etc.* In general, the host cell DNA that it is desirable to exclude from compositions of the invention is DNA that is longer than 100bp.

It is preferred that the bulk material should not require any further purification steps to be performed after its retrieval from storage. Post-storage processing steps (see below) will include dilution and packaging, but may also include the addition of components that are unsuitable for storage. For instance, if the bulk is stored in frozen form but the final vaccine is to be adjuvanted then the adjuvant will typically be added after storage. Thus the stored bulk will usually be adjuvant-free. If an adjuvant in a final vaccine is formed from several components, however, then one or more of these may be present in the stored bulk, but at least one component will be absent *e.g.* if a final vaccine is to be adjuvanted by a combination of an immunostimulatory oligonucleotide and an emulsion then the oligonucleotide may be present in the stored bulk whereas the emulsion may be absent. The choice of whether to include an adjuvant (or sub-components thereof) in the stored bulk will depend on factors such as the storage conditions (*e.g.* if freezing is involved) and the characteristics of the adjuvant (*e.g.* stability under freezing conditions), *etc.* Delaying these processing steps until after storage also advantageously allows the decision process to be delayed.

### Storage of bulk vaccine

Bulk vaccine is stored in frozen form after it has been prepared. Storage of bulk aqueous vaccine will generally be at below 0°C, *e.g.* below -10°C. Preferred freezing is at about -20°C. Deep freezing at about -80°C can also be used. Freezing between 0°C and -100°C will be typical.

The bulk vaccine is preferably stored for at least 2 months *e.g.* ≥3 months, ≥4 months, ≥5 months, ≥6 months, ≥7 months, ≥8 months, ≥9 months, ≥10 months, ≥11 months, or longer. The bulk can be stored for over a year, thus allowing stockpiling well in advance of urgent demand. Storage will typically be for less than 5 years.

### Further use of the bulk vaccine after storage

When it is required, a stored vaccine can be removed from storage and, after any necessary thawing and/or thermal equilibration, can be used for preparing final vaccines.

Typical post-storage procedures will include: addition of any further pharmaceutical components, such as adjuvants, *etc*.; dilution of the bulk to provide the desired final antigen concentration; mixing of antigens from different strains if a multivalent vaccine is desired; and filling into containers (*e.g.* vials, syringes, *etc*.)*.* Material will not usually be subjected to any further purification after storage (particularly no further antigen purification). The absence of further purification steps means that all components present in the bulk (*e.g.* detergents, non-HA antigens, *etc*.) will also be present in the final vaccines, but at lower concentrations.

The antigen concentration in a final vaccine will be lower than in the bulk vaccine. Final inactivated vaccines may include between 0.1 and 150µg of HA per influenza strain, preferably between 0.1 and 50µg *e.g.* 0.1-20µg, 0.1-15µg, 0.1-10µg, 0.1-7.5µg, 0.5-5µg, *etc.* Particular doses include *e.g.* about 15, about 10, about 7.5, about 5, about 3.8, about 1.9, about 1.5, *etc.* per strain. These lower doses are most useful when an adjuvant is present in the vaccine. Final live vaccines may have a TCID₅₀ of between 10⁶ and 10⁸. The volumes and concentrations of materials used in post-storage steps will be selected to provide these doses in the final vaccines.

If a final vaccine is to be multivalent then antigen from the stored bulk will be combined with antigen(s) from other strains. The other antigens may also have been stored according to the invention, but they may have been stored in other ways, or may have been prepared without storage.

Containers for final vaccines include vials, syringes (*e.g.* disposable syringes), nasal sprays, *etc.* These containers should be sterile.

Where a composition is located in a vial, the vial is preferably made of a glass or plastic material. The vial is preferably sterilized before the composition is added to it. To avoid problems with latex-sensitive patients, vials are preferably sealed with a latex-free stopper, and the absence of latex in all packaging material is preferred. A vial may include a single dose of vaccine, or may include more than one dose (a 'multidose' vial) *e.g.* 10 doses. Preferred vials are made of colorless glass.

A vial can have a cap (*e.g.* a Luer lock) adapted such that a pre-filled syringe can be inserted into the cap, the contents of the syringe can be expelled into the vial (*e.g.* to reconstitute lyophilised material therein), and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed. A vial may have a cap that permits aseptic removal of its contents, particularly for multidose vials.

Where a component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Preferred syringes are those marketed under the trade name "Tip-Lok"™.

Containers may be marked to show a half-dose volume *e.g.* to facilitate delivery to children. For instance, a syringe containing a 0.5ml dose may have a mark showing a 0.25ml volume.

Where a glass container (*e.g.* a syringe or a vial) is used, then it is preferred to use a container made from a borosilicate glass rather than from a soda lime glass.

A composition may be packaged (*e.g.* in the same box) with a leaflet including details of the vaccine *e.g.* instructions for administration, details of the antigens within the vaccine, *etc.* The instructions may also contain warnings *e.g.* to keep a solution of adrenaline readily available in case of anaphylactic reaction following vaccination, *etc.*

If a vaccine is adjuvanted, and if the adjuvant is included within the container, the adjuvant will generally be added in a post-storage step. Typical adjuvants for use with influenza vaccines include, but are not limited to:
- Oil-in-water emulsions (see in more detail below).
- A mineral-containing composition, including calcium salts and aluminum salts (or mixtures thereof). Calcium salts include calcium phosphate (*e.g.* the "CAP" particles disclosed in ref. 61). Aluminum salts include hydroxides, phosphates, sulfates, *etc.,* with the salts taking any suitable form (*e.g.* gel, crystalline, amorphous, *etc.*)*.* Adsorption to these salts is preferred. The mineral containing compositions may also be formulated as a particle of metal salt [62]. Aluminum salt adjuvants are described in more detail below.
- Cytokine-inducing agents (see in more detail below).
- Saponins [chapter 22 of ref. 104], which are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™. Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 63. Saponin formulations may also comprise a sterol, such as cholesterol [64]. Combinations of saponins and cholesterols can be used to form unique particles called immunostimulating complexs (ISCOMs) [chapter 23 of ref. 104]. ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 64-66. Optionally, the ISCOMS may be devoid of additional detergent [67]. A review of the development of saponin based adjuvants can be found in refs. 68 & 69.
- Bacterial ADP-ribosylating toxins (*e.g.* the *E.coli* heat labile enterotoxin "LT", cholera toxin "CT", or pertussis toxin "PT") and detoxified derivatives thereof, such as the mutant toxins known as LT-K63 and LT-R72 [70]. The use of detoxified ADP-ribosylating toxins as mucosal adjuvants is described in ref. 71 and as parenteral adjuvants in ref. 72.
- Bioadhesives and mucoadhesives, such as esterified hyaluronic acid microspheres [73] or chitosan and its derivatives [74].
- Microparticles (*i.e.* a particle of ∼100nm to ∼150µm in diameter, more preferably ∼200nm to ∼30µm in diameter, or ∼500nm to ∼10µm in diameter) formed from materials that are biodegradable and non-toxic (*e.g.* a poly(α-hydroxy acid), a polyhydroxybutyric acid, a polyorthoester, a polyanhydride, a polycaprolactone, *etc*.), with poly(lactide-co-glycolide) being preferred, optionally treated to have a negatively-charged surface (*e.g.* with SDS) or a positively-charged surface (*e.g.* with a cationic detergent, such as CTAB).
- Liposomes (Chapters 13 & 14 of ref. 104). Examples of liposome formulations suitable for use as adjuvants are described in refs. 75-77.
- Polyoxyethylene ethers and polyoxyethylene esters [78]. Such formulations further include polyoxyethylene sorbitan ester surfactants in combination with an octoxynol [79] as well as polyoxyethylene alkyl ethers or ester surfactants in combination with at least one additional non-ionic surfactant such as an octoxynol [80]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.
- Muramyl peptides, such as N-acetylmuramyl-L-threonyl-D-isoglutamine ("thr-MDP"), N-acetyl-normuramyl-L-alanyl-D-isoglutamine (nor-MDP), N-acetylglucsaminyl-N-acetylmuramyl-L-Al-D-isoglu-L-Ala-dipaimitoxy propylamide ("DTP-DPP", or "Theramide™), N-acetylmuramyl-L-alanyl-D-isoglutaminyl-L-alanine-2-(1'-2'dipalmitoyl-sn-glycero-3-hydroxyphosphoryloxy)-ethylamine ("MTP-PE").
- An outer membrane protein proteosome preparation prepared from a first Gram-negative bacterium in combination with a liposaccharide preparation derived from a second Gram-negative bacterium, wherein the outer membrane protein proteosome and liposaccharide preparations form a stable non-covalent adjuvant complex. Such complexes include "IVX-908", a complex comprised of *Neisseria meningitidis* outer membrane and lipopolysaccharides. They have been used as adjuvants for influenza vaccines [81].
- A polyoxidonium polymer [82,83] or other N-oxidized polyethylene-piperazine derivative.
- Methyl inosine 5'-monophosphate ("MIMP") [84].
- A polyhydroxiated pyrrolizidine compound [85], such as one having formula: 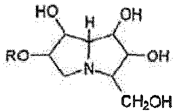 where R is selected from the group comprising hydrogen, straight or branched, unsubstituted or substituted, saturated or unsaturated acyl, alkyl (*e.g.* cycloalkyl), alkenyl, alkynyl and aryl groups, or a pharmaceutically acceptable salt or derivative thereof. Examples include, but are not limited to: casuarine, casuarine-6-α-D-glucopyranose, 3-epi-casuarine, 7-*epi*-casuarine, 3,7-di*epi*-casuarine, *etc.*
- A CD1d ligand, such as an α-glycosylceramide [86-93] (*e.g.* α-galactosylceramide), phytosphingosine-containing α-glycosylceramides, OCH, KRN7000 [(2S,3S,4R)-1-O-(α-D-galactopyranosyl)-2-(N-hexacosanoylamino)-1,3,4-octadecanetriol], CRONY-101, 3"-O-sulfo-galactosylceramide, *etc.*
- A gamma inulin [94] or derivative thereof, such as algammulin.
- A compound of formula I, II or III, or a salt thereof: 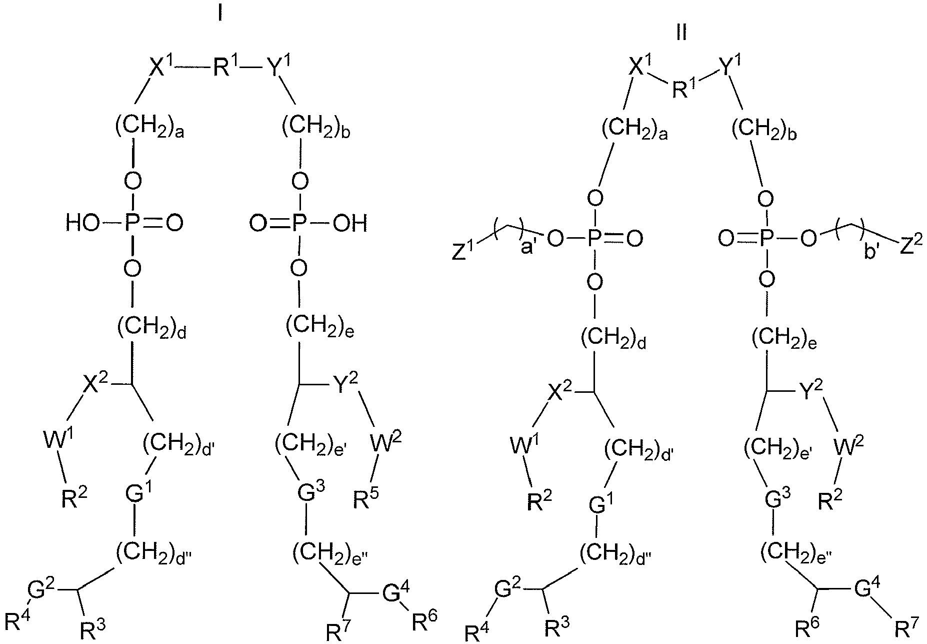 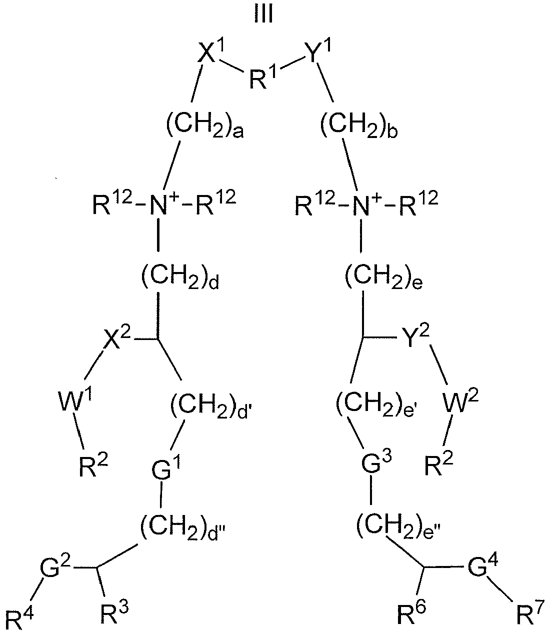 as defined in reference 95, such as 'ER 803058', 'ER 803732', 'ER 804053', ER 804058', 'ER 804059', 'ER 804442', 'ER 804680', 'ER 804764', ER 803022 or 'ER 804057' *e.g.*: 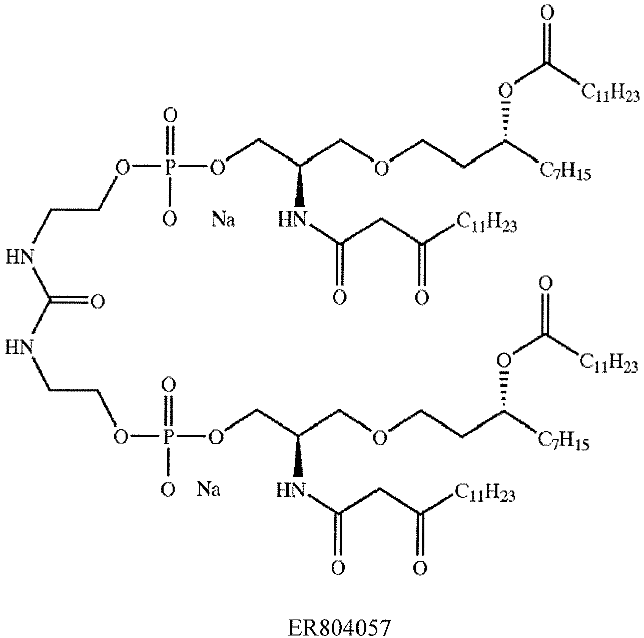 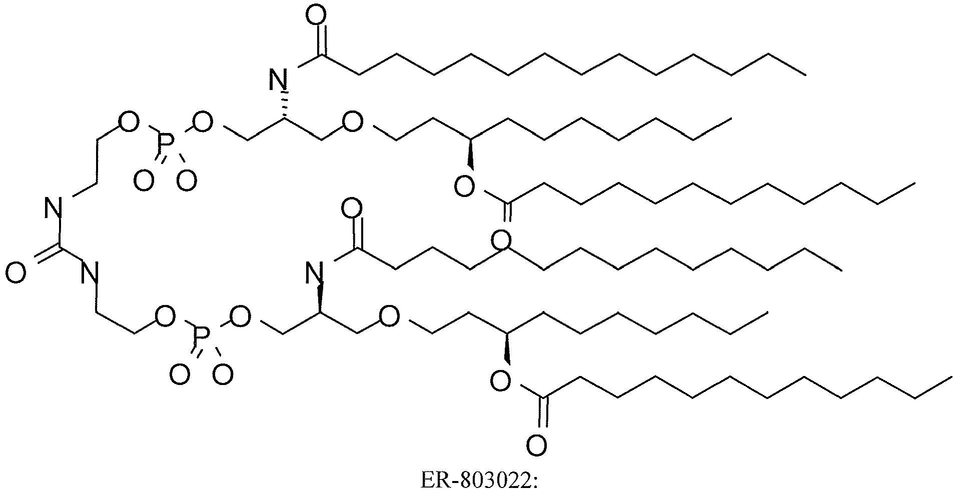
- Derivatives of lipid A from *Escherichia coli* such as OM-174 (described in refs. 96 & 97).
- A formulation of a cationic lipid and a (usually neutral) co-lipid, such as aminopropyl-dimethyl-myristoleyloxy-propanaminium bromide-diphytanoylphosphatidyl-ethanolamine ("Vaxfectin™") or aminopropyl-dimethyl-bis-dodecyloxy-propanaminium bromide-dioleoylphosphatidyl-ethanolamine ("GAP-DLRIE:DOPE"). Formulations containing (±)-N-(3-aminopropyl)-N,N-dimethyl-2,3-bis(syn-9-tetradeceneyloxy)-1-propanaminium salts are preferred [98].
- 3-O-deacylated monophosphoryl lipid A (see above).
- Compounds containing lipids linked to a phosphate-containing acyclic backbone, such as the TLR4 antagonist E5564 [99,100]:

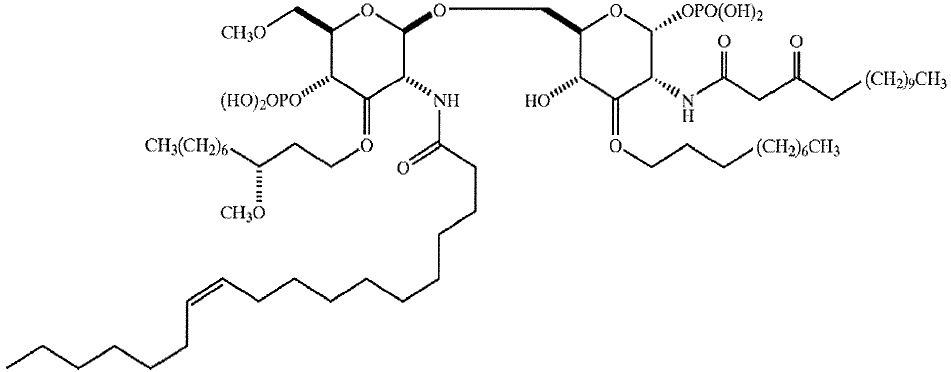

These and other adjuvant-active substances are discussed in more detail in references 104 & 105.

Compositions may include two or more of said adjuvants. For example, they may advantageously include both an oil-in-water emulsion and a cytokine-inducing agent, as this combination improves the cytokine responses elicited by influenza vaccines, such as the interferon-γ response, with the improvement being much greater than seen when either the emulsion or the agent is used on its own.

Antigens and adjuvants in a composition will typically be in admixture.

### Oil-in-water emulsion adjuvants

Oil-in-water emulsions have been found to be particularly suitable for use in adjuvanting influenza virus vaccines. Various such emulsions are known, and they typically include at least one oil and at least one surfactant, with the oil(s) and surfactant(s) being biodegradable (metabolisable) and biocompatible. The oil droplets in the emulsion are generally less than 5µm in diameter, and may even have a sub-micron diameter, with these small sizes being achieved with a microfluidiser to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g.* obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Non-ionic surfactants are preferred. Preferred surfactants for including in the emulsion are Tween 80 (polyoxyethylene sorbitan monooleate), Span 85 (sorbitan trioleate), lecithin and Triton X-100.

Mixtures of surfactants can be used *e.g.* Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [101-103], as described in more detail in Chapter 10 of ref. 104 and chapter 12 of ref. 105. The MF59 emulsion advantageously includes citrate ions *e.g.* 10mM sodium citrate buffer.
- An emulsion of squalene, a tocopherol, and Tween 80. The emulsion may include phosphate buffered saline. It may also include Span 85 (*e.g.* at 1%) and/or lecithin. These emulsions may have from 2 to 10% squalene, from 2 to 10% tocopherol and from 0.3 to 3% Tween 80, and the weight ratio of squaleneaocopherol is preferably ≤1 as this provides a more stable emulsion. Squalene and Tween 80 may be present volume ratio of about 5:2. For instance, an emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g.* with an average diameter of between 100 and 250nm, preferably about 180nm.
- An emulsion of squalene, a tocopherol, and a Triton detergent (*e.g.* Triton X-100). The emulsion may also include a 3d-MPL (see below). The emulsion may contain a phosphate buffer.
- An emulsion comprising a polysorbate (*e.g.* polysorbate 80), a Triton detergent (*e.g.* Triton X-100) and a tocopherol (*e.g.* an α-tocopherol succinate). The emulsion may include these three components at a mass ratio of about 75:11:10 (*e.g.* 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [106] (0.05-1% Thr-MDP, 5% squalane, 2.5% Pluronic L121 and 0.2% polysorbate 80). It can also be used without the Thr-MDP, as in the "AF" adjuvant [107] (5% squalane, 1.25% Pluronic L121 and 0.2% polysorbate 80). Microfluidisation is preferred.
- An emulsion having from 0.5-50% of an oil, 0.1-10% of a phospholipid, and 0.05-5% of a non-ionic surfactant. As described in reference 108, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 109, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin (*e.g.* QuilA or QS21) and a sterol (*e.g.* a cholesterol) are associated as helical micelles [110].
- An emulsion comprising a mineral oil, a non-ionic lipophilic ethoxylated fatty alcohol, and a non-ionic hydrophilic surfactant (*e.g*. an ethoxylated fatty alcohol and/or polyoxyethylene-polyoxypropylene block copolymer) [111].
- An emulsion comprising a mineral oil, a non-ionic hydrophilic ethoxylated fatty alcohol, and a non-ionic lipophilic surfactant (*e.g.* an ethoxylated fatty alcohol and/or polyoxyethylene-polyoxypropylene block copolymer) [111].

Emulsions will generally be added to a liquid antigen preparation. The volume ratio of the two liquids for mixing can vary (*e.g.* between 5:1 and 1:5) but is generally about 1:1.

After the antigen and adjuvant have been mixed, haemagglutinin antigen will generally remain in aqueous solution but may distribute itself around the oil/water interface. In general, little if any haemagglutinin will enter the oil phase of the emulsion.

Where a composition includes a tocopherol, any of the α, β, γ, δ, ε or ξ tocopherols can be used, but α-tocopherols are preferred. The tocopherol can take several forms *e.g.* different salts and/or isomers. Salts include organic salts, such as succinate, acetate, nicotinate, *etc.* D-α-tocopherol and DL-α-tocopherol can both be used. Tocopherols are advantageously included in vaccines for use in elderly patients (*e.g.* aged 60 years or older) because vitamin E has been reported to have a positive effect on the immune response in this patient group [112]. They also have antioxidant properties that may help to stabilize the emulsions [113]. A preferred α-tocopherol is DL-α-tocopherol, and the preferred salt of this tocopherol is the succinate. The succinate salt has been found to cooperate with TNF-related ligands *in vivo.* Moreover, α-tocopherol succinate is known to be compatible with influenza vaccines and to be a useful preservative as an alternative to mercurial compounds [46].

### Cytokine-inducing agents

Cytokine-inducing agents for inclusion in compositions of the invention are able, when administered to a patient, to elicit the immune system to release cytokines, including interferons and interleukins. Cytokine responses are known to be involved in the early and decisive stages of host defense against influenza infection [114]. Preferred agents can elicit the release of one or more of: interferon-γ; interleukin-1; interleukin-2; intsrleukin-12; TNF-α: TNF-β; and GM-CSF. Preferred agents elicit the release of cytokines associated with a Th1-type immune response *e.g.* interferon-γ, TNF-α, interleukin-2. Stimulation of both interferon-γ and interleukin-2 is preferred.

As a result of receiving a composition of the invention, therefore, a patient will have T cells that, when stimulated with an influenza antigen, will release the desired cytokine(s) in an antigen-specific manner. For example, T cells purified form their blood will release γ-interferon when exposed in *vitro* to influenza virus haemagglutinin. Methods for measuring such responses in peripheral blood mononuclear cells (PBMC) are known in the art, and include ELISA, ELISPOT, flow-cytometry and real-time PCR. For example, reference 115 reports a study in which antigen-specific T cell-mediated immune responses against tetanus toxoid, specifically γ-interferon responses, were monitored, and found that ELISPOT was the most sensitive method to discriminate antigen-specific TT-induced responses from spontaneous responses, but that intracytoplasmic cytokine detection by flow cytometry was the most efficient method to detect re-stimulating effects.

Suitable cytokine-inducing agents include, but are not limited to:
- An immunostimulatory oligonucleotide, such as one containing a CpG motif (a dinucleotide sequence containing an unmethylated cytosine linked by a phosphate bond to a guanosine), or a double-stranded RNA, or an oligonucleotide containing a palindromic sequence, or an oligonucleotide containing a poly(dG) sequence.
- 3-O-deacylated monophosphoryl lipid A ('3dMPL', also known as 'MPL™') [116-119].
- An imidazoquinoline compound, such as Imiquimod ("R-837") [120,121], Resiquimod ("R-848") [122], and their analogs; and salts thereof (*e.g.* the hydrochloride salts). Further details about immunostimulatory imidazoquinolines can be found in references 123 to 127.
- A thiosemicarbazone compound, such as those disclosed in reference 128. Methods of formulating, manufacturing, and screening for active compounds are also described in reference 128. The thiosemicarbazones are particularly effective in the stimulation of human peripheral blood mononuclear cells for the production of cytokines, such as TNF-α.
- A tryptanthrin compound, such as those disclosed in reference 129. Methods of formulating, manufacturing, and screening for active compounds are also described in reference 129. The thiosemicarbazones are particularly effective in the stimulation of human peripheral blood mononuclear cells for the production of cytokines, such as TNF-α.
- A nucleoside analog, such as: (a) Isatorabine (ANA-245; 7-thia-8-oxoguanosine): 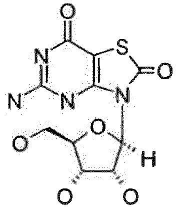 and prodrugs thereof; (b) ANA975; (c) ANA-025-1; (d) ANA380; (e) the compounds disclosed in references 130 to 132; (f) a compound having the formula: 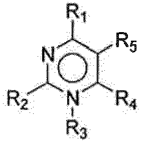 wherein:
   R₁ and R₂ are each independently H, halo, -NRₐR_{b}, -OH, C₁₋₆ alkoxy, substituted C₁₋₆ alkoxy, heterocyclyl, substituted heterocyclyl, C₆₋₁₀ aryl, substituted C₆₋₁₀ aryl, C₁₋₆ alkyl, or substituted C₁₋₆ alkyl;
   R₃ is absent, H, C₁₋₆ alkyl, substituted C₁₋₆ alkyl, C₆₋₁₀ aryl, substituted C₆₋₁₀ aryl, heterocyclyl, or substituted heterocyclyl;
   R₄ and R₅ are each independently H, halo, heterocyclyl, substituted heterocyclyl, -C(O)-R_{d}, C₁₋₆ alkyl, substituted C₁₋₆ alkyl, or bound together to form a 5 membered ring as in R₄₋₅: 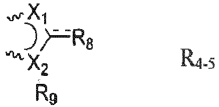 the binding being achieved at the bonds indicated by a 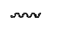
   X₁ and X₂ are each independently N, C, O, or S;
   R₈ is H, halo, -OH, C₁₋₆ alkyl, C₂₋₆ alkenyl, C₂₋₆ alkynyl, -OH, -NRₐR_{b}, -(CH₂)ₙ-O-R_{c}, -O-(C₁₋₆ alkyl), -S(O)ₚRₑ, or -C(O)-R_{d};
   R₉ is H, C₁₋₆ alkyl, substituted C₁₋₆ alkyl, heterocyclyl, substituted heterocyclyl or R₉ₐ, wherein R₉ₐ is: 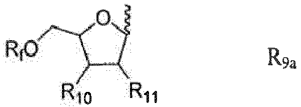 the binding being achieved at the bond indicated by a 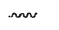
   R₁₀ and R₁₁ are each independently H, halo, C₁₋₆ alkoxy, substituted C₁₋₆ alkoxy, - NRₐR_{b}, or -OH;
   each Rₐ and R_{b} is independently H, C₁₋₆ alkyl, substituted C₁₋₆ alkyl, -C(O)R_{d}, C₆₋₁₀ aryl; each R_{c} is independently H, phosphate, diphosphate, triphosphate, C₁₋₆ alkyl, or substituted C₁₋₆ alkyl;
   each R_{d} is independently H, halo, C₁₋₆ alkyl, substituted C₁₋₆ alkyl, C₁₋₆ alkoxy, substituted C₁₋₆ alkoxy, -NH₂, -NH(C₁₋₆ alkyl), -NH(substituted C₁₋₆ alkyl), -N(C₁₋₆ alkyl)₂, -N(substituted C₁₋₆ alkyl)₂, C₆₋₁₀ aryl, or heterocyclyl;
   each Rₑ is independently H, C₁₋₆ alkyl, substituted C₁₋₆ alkyl, C₆₋₁₀ aryl, substituted C₆₋₁₀ aryl, heterocyclyl, or substituted heterocyclyl;
   each R_{f} is independently H, C₁₋₆ alkyl, substituted C₁₋₆ alkyl, -C(O)R_{d}, phosphate, diphosphate, or triphosphate;
   each n is independently 0, 1, 2, or 3;
   each p is independently 0, 1, or 2; or
   or (g) a pharmaceutically acceptable salt of any of (a) to (f), a tautomer of any of (a) to (f), or a pharmaceutically acceptable salt of the tautomer.
- Loxoribine (7-allyl-8-oxoguanosine) [133].
- Compounds disclosed in reference 134, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [135,136], Hydrapthalamide compounds, Benzophenone compounds, Isoxazole compounds, Sterol compounds, Quinazilinone compounds, Pyrrole compounds [137], Anthraquinone compounds, Quinoxaline compounds, Triazine compounds, Pyrazalopyrimidine compounds, and Benzazole compounds [138].
- Compounds disclosed in reference 139.
- An aminoalkyl glucosaminide phosphate derivative, such as RC-529 [140,141].
- A phosphazene, such as poly[di(carboxylatophenoxy)phosphazene] ("PCPP") as described, for example, in references 142 and 143.
- Small molecule immunopotentiators (SMIPs) such as:
   N2-methyl-1-(2-methylpropyl)-1H-imidazo[4,5-c]quinoline-2,4-diamine N2,N2-dimethyl-1-(2-methylpropyl)-1H-imidazo[4,5-c]quinoline-2,4-diamine N2-ethyl-N2-methyl-1-(2-methylpropyl)-1H-imidazo[4,5-c]quinoline-2,4-diamine N2-methyl-1-(2-methylpropyl)-N2-propyl-1H-imidazo[4,5-c]quinoline-2,4-diamine 1-(2-methylpropyl)-N2-propyl-1H-imidazo[4,5-c]quinoline-2,4-diamine N2-butyl-1-(2-methylpropyl)-1H-imidazo[4,5-c]quinoline-2,4-diamine N2-butyl-N2-methyl-1-(2-methylpropyl)-1H-imidazo[4,5-c]quinoline-2,4-diamine N2-methyl-1-(2-methylpropyl)-N2-pentyl-1H-imidazo[4,5-c]quinoline-2,4-diamine N2-methyl-1-(2-methylpropyl)-N2-prop-2-enyl-1H-imidazo[4,5-c]quinoline-2,4-diamine 1-(2-methylpropyl)-2-[(phenylmethyl)thio]-1H-imidazo[4,5-c]quinolin-4-amine 1-(2-methylpropyl)-2-(propylthio)-1H-imidazo[4,5-c]quinolin-4-amine 2-[[4-amino-1-(2-methylpropyl)-1H-imidazo[4,5-c]quinolin-2-yl](methyl)amino]ethanol 2-[[4-amino-1-(2-methylpropyl)-1H-imidazo[4,5-c]quinoliii-2-yi](methyl)amino]ethyl acetate 4-amino-1-(2-methylpropyl)-1,3-dihydro-2H-imidazo[4,5-c]quinolin-2-one
   N2-butyl-1-(2-methylpropyl)-N4,N4-bis(phenylmethyl)-1H-imidazo[4,5-c]quinoline-2,4-diamine
   N2-butyl-N2-methyl-1-(2-methylpropyl)-N4,N4-bis(phenylmethyl)-1H-imidazo[4,5-c]quinoline-2,4-diamine
   N2-methyl-1-(2-methylpropyl)-N4,N4-bis(phenylmethyl)-1H-imidazo[4,5-c]quinoline-2,4-diamine
   N2,N2-dimethyl-1-(2-methylpropyl)-N4,N4-bis(phenylmethyl)-1H-imidazo[4,5-c]quinoline-2,4-diamine
   1-{4-amino-2-[methyl(propyl)amino]-1H-imidazo[4,5-c]quinolin-1-yl}-2-methylpropan-2-ol 1-[4-amino-2-(propylamino)-1H-imidazo[4,5-c]quinolin-1-yl]-2-methylpropan-2-ol N4,N4-dibenzyl-1-(2-methoxy-2-methylpropyl)-N2-propyl-1H-imidazo[4,5-c]quinoline-2,4-diamine.

The cytokine-inducing agents for use in the present invention may be modulators and/or agonists of Toll-Like Receptors (TLR). For example, they may be agonists of one or more of the human TLR1, TLR2, TLR3, TLR4, TLR7, TLR8, and/or TLR9 proteins. Preferred agents are agonists of TLR7 (*e.g.* imidazoquinolines) and/or TLR9 (*e.g.* CpG oligonucleotides). These agents are useful for activating innate immunity pathways.

The cytokine-inducing agent can be added to the composition at various stages during its production. For example, it may be within an antigen composition, and this mixture can then be added to an oil-in-water emulsion. As an alternative, it may be within an oil-in-water emulsion, in which case the agent can either be added to the emulsion components before emulsification, or it can be added to the emulsion after emulsification. Similarly, the agent may be coacervated within the emulsion droplets. The location and distribution of the cytokine-inducing agent within the final composition will depend on its hydrophilic/lipophilic properties *e.g.* the agent can be located in the aqueous phase, in the oil phase, and/or at the oil-water interface.

The cytokine-inducing agent can be conjugated to a separate agent, such as an antigen (*e.g.* CRM197). A general review of conjugation techniques for small molecules is provided in ref. 144. As an alternative, the adjuvants may be non-covalently associated with additional agents, such as by way of hydrophobic or ionic interactions.

Two preferred cytokine-inducing agents are (a) immunostimulatory oligonucleotides and (b) 3dMPL. Immunostimulatory oligonucleotides can include nucleotide modifications/analogs such as phosphorothioate modifications and can be double-stranded or (except for RNA) single-stranded. References 145, 146 and 147 disclose possible analog substitutions *e.g.* replacement of guanosine with 2'-deoxy-7-deazaguanosine. The adjuvant effect of CpG oligonucleotides is further discussed in refs. 148-153. A CpG sequence may be directed to TLR9, such as the motif GTCGTT or TTCGTT [154]. The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in refs. 155-157. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, references 154 & 158-160. A useful CpG adjuvant is CpG7909, also known as ProMune™ (Coley Pharmaceutical Group, Inc.).

As an alternative, or in addition, to using CpG sequences, TpG sequences can be used [161]. These oligonucleotides may be free from unmethylated CpG motifs.

The immunostimulatory oligonucleotide may be pyrimidine-rich. For example, it may comprise more than one consecutive thymidine nucleotide (*e.g*. TTTT, as disclosed in ref. 161), and/or it may have a nucleotide composition with >25% thymidine (*e.g*. >35%, >40%, >50%, >60%, >80%, *etc.*). For example, it may comprise more than one consecutive cytosine nucleotide (*e.g*. CCCC, as disclosed in ref. 161), and/or it may have a nucleotide composition with >25% cytosine (*e.g*. >35%, >40%, >50%, >60%, >80%, *etc.*). These oligonucleotides may be free from unmethylated CpG motifs. Immunostimulatory oligonucleotides will typically comprise at least 20 nucleotides. They may comprise fewer than 100 nucteotides,

3dMPL (also known as 3 de-O-acylated monophosphoryl lipid A or 3-O-desacyl-4'-monophosphoryl lipid A) is an adjuvant in which position 3 of the reducing end glucosamine in monophosphoryl lipid A has been de-acylated. 3dMPL has been prepared from a heptoseless mutant of *Salmonella minnesota,* and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF (see also ref. 162). Preparation of 3dMPL was originally described in reference 163, and the product has been manufactured and sold by Corixa Corporation under the name MPL™. Further details can be found in refs 116 to 119.

3dMPL can take the form of a mixture of related molecules, varying by their acylation (*e.g*. having 3, 4, 5 or 6 acyl chains, which may be of different lengths). The two glucosamine (also known as 2-deoxy-2-amino-glucose) monosaccharides are N-acylated at their 2-position carbons (*i.e*. at positions 2 and 2'), and there is also O-acylation at the 3' position. The group attached to carbon 2 has formula -NH-CO-CH₂-CR¹R^{1'}. The group attached to carbon 2' has formula -NH-CO-CH₂-CR²R^{2'}. The group attached to carbon 3' has formula -O-CO-CH₂-CR³R^{3'}. A representative structure is:

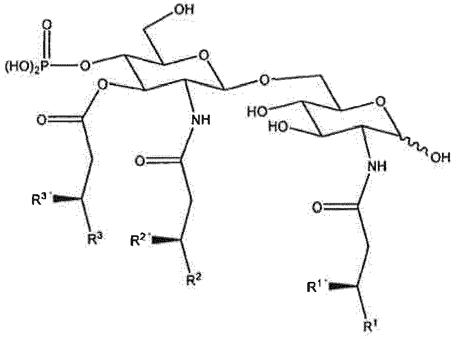

Groups R¹, R² and R³ are each independently -(CH₂)ₙ-CH₃. The value of *n* is preferably between 8 and 16, more preferably between 9 and 12, and is most preferably 10.

Groups R^{1'}, R^{2'}and R^{3'} can each independently be: (a) -H; (b) -OH; or (c) -O-CO-R⁴,where R⁴ is either -H or -(CH₂)ₘ-CH₃, wherein the value of m is preferably between 8 and 16, and is more preferably 10, 12 or 14. At the 2 position, *m* is preferably 14. At the 2' position, *m* is preferably 10. At the 3' position, m is preferably 12. Groups R^{1'}, R^{2'} and R^{3'} are thus preferably -O-acyl groups from dodecanoic acid, tetradecanoic acid or hexadecanoic acid.

When all of R^{1'}, R^{2'} and R^{3'} are -H then the 3dMPL has only 3 acyl chains (one on each of positions 2, 2' and 3'). When only two of R^{1'}, R^{2'} and R^{3'} are -H then the 3dMPL can have 4 acyl chains. When only one of R^{1'}, R^{2'} and R^{3'} is -H then the 3dMPL can have 5 acyl chains. When none of R^{1'}, R^{2'} and R^{3'} is -H then the 3dMPL can have 6 acyl chains. The 3dMPL adjuvant used according to the invention can be a mixture of these forms, with from 3 to 6 acyl chains, but it is preferred to include 3dMPL with 6 acyl chains in the mixture, and in particular to ensure that the hexaacyl chain form makes up at least 10% by weight of the total 3dMPL *e.g*. ≥20%, ≥30%, ≥40%, ≥50% or more. 3dMPL with 6 acyl chains has been found to be the most adjuvant-active form.

Thus the most preferred form of 3dMPL for inclusion in compositions of the invention is:

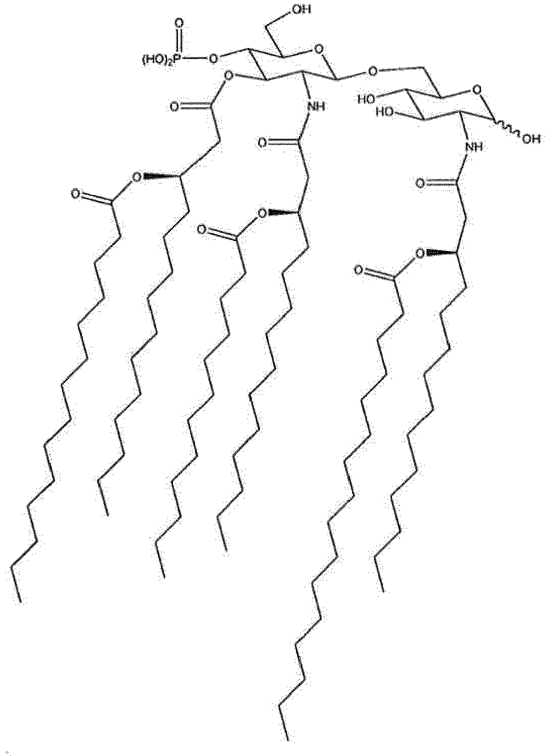

Where 3dMPL is used in the form of a mixture then references to amounts or concentrations of 3dMPL in compositions of the invention refer to the combined 3dMPL species in the mixture.

In aqueous conditions, 3dMPL can form micellar aggregates or particles with different sizes *e.g*. with a diameter <150nm or >500nm. Either or both of these can be used with the invention, and the better particles can be selected by routine assay. Smaller particles (*e.g*. small enough to give a clear aqueous suspension of 3dMPL) are preferred for use according to the invention because of their superior activity [164]. Preferred particles have a mean diameter less than 220nm, more preferably less than 200nm or less than 150nm or less than 120nm, and can even have a mean diameter less than 100nm. In most cases, however, the mean diameter will not be lower than 50nm. These particles are small enough to be suitable for filter sterilization. Particle diameter can be assessed by the routine technique of dynamic light scattering, which reveals a mean particle diameter. Where a particle is said to have a diameter ofx nm, there will generally be a distribution of particles about this mean, but at least 50% by number (*e.g*. ≥60%, ≥70%, ≥80%, ≥90%, or more) of the particles will have a diameter within the range *x*±25%.

3dMPL can advantageously be used in combination with an oil-in-water emulsion. Substantially all of the 3dMPL may be located in the aqueous phase of the emulsion.

The 3dMPL can be used on its own, or in combination with one or more further compounds. For example, it is known to use 3dMPL in combination with the QS21 saponin [165] (including in an oil-in-water emulsion [166]), with an immunostimulatory oligonucleotide, with both QS21 and an immunostimulatory oligonucleotide, with aluminum phosphate [167], with aluminum hydroxide [168], or with both aluminum phosphate and aluminum hydroxide.

### Aluminum salt adjuvants,

The adjuvants known as aluminum hydroxide and aluminum phosphate may be used. These names are conventional, but are used for convenience only, as neither is a precise description of the actual chemical compound which is present (*e.g*. see chapter 9 of reference 104). The invention can use any of the "hydroxide" or "phosphate" adjuvants that are in general use as adjuvants.

The adjuvants known as "aluminium hydroxide" are typically aluminium oxyhydroxide salts, which are usually at least partially crystalline. Aluminium oxyhydroxide, which can be represented by the formula AlO(OH), can be distinguished from other aluminium compounds, such as aluminium hydroxide Al(OH)₃, by infrared (IR) spectroscopy, in particular by the presence of an adsorption band at 1070cm⁻¹ and a strong shoulder at 3090-3100cm⁻¹, [chapter 9 of ref. 104]. The degree of crystallinity of an aluminium hydroxide adjuvant is reflected by the width of the diffraction band at half height (WHH), with poorly-crystalline particles showing greater line broadening due to smaller crystallite sizes. The surface area increases as WHH increases, and adjuvants with higher WHH values have been seen to have greater capacity for antigen adsorption. A fibrous morphology (*e.g*. as seen in transmission electron micrographs) is typical for aluminium hydroxide adjuvants. The pl of aluminium hydroxide adjuvants is typically about 11 *i.e.* the adjuvant itself has a positive surface charge at physiological pH. Adsorptive capacities of between 1.8-2.6 mg protein per mg Al⁺⁺⁺ at pH 7.4 have been reported for aluminium hydroxide adjuvants.

The adjuvants known as "aluminium phosphate" are typically aluminium hydroxyphosphates, often also containing a small amount of sulfate (*i.e*. aluminium hydroxyphosphate sulfate). They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 1.2. Hydroxyphosphates can be distinguished from strict AlPO₄ by the presence of hydroxyl groups. For example, an IR spectrum band at 3164cm⁻¹ (*e.g*. when heated to 200°C) indicates the presence of structural hydroxyls [ch.9 of ref. 104]

The PO₄/Al³⁺ molar ratio of an aluminium phosphate adjuvant will generally be between 0.3 and 1.2, preferably between 0.8 and 1.2, and more preferably 0.95+0.1. The aluminium phosphate will generally be amorphous, particularly for hydroxyphosphate salts. A typical adjuvant is amorphous aluminium hydroxyphosphate with PO₄/Al molar ratio between 0.84 and 0.92, included at 0.6mg Al³⁺/ml. The aluminium phosphate will generally be particulate (*e.g*. plate-like morphology as seen in transmission electron micrographs). Typical diameters of the particles are in the range 0.5-20µm (*e.g*. about 5-10µm) after any antigen adsorption. Adsorptive capacities of between 0.7-1.5 mg protein per mg Al⁺⁺⁺ at pH 7.4 have been reported for aluminium phosphate adjuvants.

The point of zero charge (PZC) of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 *e.g*. about 5.7.

Suspensions of aluminium salts used to prepare compositions of the invention may contain a buffer (*e.g*. a phosphate or a histidine or a Tris buffer), but this is not always necessary. The suspensions are preferably sterile and pyrogen-free. A suspension may include free aqueous phosphate ions *e.g*. present at a concentration between 1.0 and 20 mM, preferably between 5 and 15 mM, and more preferably about 10 mM. The suspensions may also comprise sodium chloride.

The invention can use a mixture of both an aluminium hydroxide and an aluminium phosphate [49]. In this case there may be more aluminium phosphate than hydroxide *e.g.* a weight ratio of at least 2:1 *e.g.* ≥5:1, ≥6:1, ≥7:1, ≥8:1, ≥9:1, *etc.*

The concentration of Al⁺⁺⁺ in a composition for administration to a patient is preferably less than 10mg/ml *e.g.* ≤5 mg/ml, ≤4 mg/ml, ≤3 mg/ml, ≤2 g/ml, ≤1 mg/ml, *etc.* A preferred range is between 0.3 and 1mg/ml. A maximum of<0.85mg/dose is preferred.

As well as including one or more aluminium salt adjuvants, the adjuvant component may include one or more further adjuvant or immunostimulating agents. Such additional components include, but are not limited to: a 3-O-deacylated monophosphoryl lipid A adjuvant ('3d-MPL'); and/or an oil-in-water emulsion.

### Pharmaceutical compositions

Final vaccines of the invention are pharmaceutically acceptable. They may include components in addition to the influenza antigen *e.g.* they will typically include one or more pharmaceutical carrier(s) and/or excipient(s). A thorough discussion of such components is available in ref. 169.

The composition may include preservatives such as thiomersal or 2-phenoxyethanol. It is preferred, however, that the vaccine should be substantially free from (*i.e.* less than 5µg/ml) mercurial material *e.g*. thiomersal-free [46,170]. Vaccines containing no mercury are more preferred. Preservatives may be added to a bulk vaccine before freezing and/or after thawing. Preservative-free vaccines are particularly preferred.

To control tonicity, it is preferred to include a physiological salt, such as a sodium salt. Sodium chloride (NaCl]) is preferred, which may be present at between 1 and 20 mg/ml. Other salts that may be present include potassium chloride, potassium dihydrogen phosphate, disodium phosphate dehydrate, magnesium chloride, calcium chloride, *etc.* These salts may be added to a bulk vaccine before freezing and/or after thawing. Adding before freezing is preferred, and potassium dihydrogen phosphate is particularly useful for material which will be frozen.

Final vaccines may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range. Buffering substances may be added to a bulk vaccine before freezing and/or after thawing. Adding before freezing is preferred, and a phosphate buffer is particularly useful for material which will be frozen.

Final vaccines will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [171], but keeping osmolality in this range is nevertheless preferred.

The pH of a final vaccine will generally be between 5.0 and 8.1, and more typically between 6.0 and *8.0 e.g*. between 6.5 and 7.5, or between 7.0 and 7.8. pH may be adjusted in a bulk vaccine before freezing and/or after thawing. Adjustment before freezing is preferred.

Final vaccines are preferably sterile. They are preferably non-pyrogenic *e.g*. containing <1 EU (endotoxin unit, a standard measure) per dose, and preferably <0.1 EU per dose. They are preferably gluten free.

Final vaccines may include material for a single immunisation, or may include material for multiple immunisations (*i.e.* a 'multidose' kit). The inclusion of a preservative is preferred in multidose arrangements and/or, as mentioned above, an aseptic cap can be used.

Influenza vaccines are typically administered in a dosage volume of about 0.5ml, although a half dose (*i.e.* about 0.25ml) may be administered to children.

Final vaccine compositions are preferably stored at between 2°C and 8°C. Unlike the stored bulk, they should not be frozen. They should ideally be kept out of direct light.

### Methods of treatment, and administration of the vaccine

Compositions of the invention are suitable for administration to human patients, and the invention provides a method of raising an immune response in a patient, comprising the step of administering a vaccine to the patient, where the vaccine was prepared using a method of the invention.

The invention also provides a vaccine prepared according to the invention, for use as a medicament.

The invention also provides the use of a frozen bulk influenza antigen preparation in the manufacture of a medicament for raising an immune response in a patient.

The immune response raised by these methods and uses will generally include an antibody response, preferably a protective antibody response. Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [172]. Antibody responses are typically measured by hemagglutination inhibition, by microneutralisation, by single radial immunodiffusion (SRID), and/or by single radial hemolysis (SRH). These assay techniques are well known in the art.

Compositions of the invention can be administered in various ways. The most preferred immunisation route is by intramuscular injection (*e.g*. into the arm or leg), but other available routes include subcutaneous injection, intranasal [173-175], oral [176], intradermal [177,178], transcutaneous, transdermal [179], *etc.*

Vaccines prepared according to the invention may be used to treat both children and adults. Influenza vaccines are currently recommended for use in pediatric and adult immunisation, from the age of 6 months. Thus the patient may be less than 1 year old, 1-5 years old, 5-15 years old, 15-55 years old, or at least 55 years old. Preferred patients for receiving the vaccines are the elderly (*e.g*. ≥50 years old, ≥60 years old, preferably ≥65 years), the young (*e.g*. ≤5 years old), hospitalised patients, healthcare workers, armed service and military personnel, pregnant women, the chronically ill, immunodeficient patients, patients who have taken an antiviral compound (*e.g*. an oseltamivir or zanamivir compound; see below) in the 7 days prior to receiving the vaccine, people with egg allergies and people travelling abroad. The vaccines are not suitable solely for these groups, however, and may be used more generally in a population. For pandemic strains, administration to all age groups is preferred.

Preferred compositions satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% seroprotection; (2)≥30% seroconversion; and/or (3) a GMT increase of ≥2-fold. These criteria are based on open label studies with at least 50 patients.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Administration of more than one dose (typically two doses) is particularly useful in immunologically naïve patients *e.g*. for people who have never received an influenza vaccine before, or for vaccinating against a new HA subtype (as in a pandemic outbreak). Multiple doses will typically be administered at least 1 week apart (*e.g*. about 2 weeks, about 3 weeks, about 4 weeks, about 6 weeks, about 8 weeks, about 12 weeks, about 16 weeks, *etc.).*

Vaccines of the invention may be administered to patients at substantially the same time as (*e.g.* during the same medical consultation or visit to a healthcare professional or vaccination centre) other vaccines *e.g*. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, vaccines of the invention may be administered to patients at substantially the same time as (*e.g*. during the same medical consultation or visit to a healthcare professional) an antiviral compound, and in particular an antiviral compound active against influenza virus (*e.g*. oseltamivir and/or zanamivir). These antivirals include neuraminidase inhibitors, such as a (3R,4R,5S)-4-acetylamino-5-amino-3(1-ethylpropoxy)-1-cyclohexene-1-carboxylic acid or 5-(acetylamino)-4-[(aminoiminomethyl)-amino]-2,6-anhydro-3,4,5-trideoxy-D-glycero-D-galactonon-2-enonic acid, including esters thereof (*e.g*. the ethyl esters) and salts thereof (*e.g*. the phosphate salts). A preferred antiviral is (3R,4R,5S)-4-acetylamino-5-amino-3(1-ethylpropoxy)-1-cyciohexene-1-carboxylic acid, ethyl ester, phosphate (1:1), also known as oseltamivir phosphate (TAMlFLU™).

### General

The term "comprising" encompasses "including" as well as "consisting" *e.g*. a composition "comprising" X may consist exclusively of X or may include something additional *e.g*. X + Y.

The word "substantially" does not exclude "completely" *e.g*. a composition which is "substantially free" from Y may be completely free from Y. Where necessary, the word "substantially" may be omitted from the definition of the invention.

The term "about" in relation to a numerical value x means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc*.

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

Where a cell substrate is used for reassortment or reverse genetics procedures, it is preferably one that has been approved for use in human vaccine production *e.g*. as in Ph Eur general chapter 5.2.3.

Bulk preparation and/or storage and/or downstream processing steps may take place within the same country or within different countries. For instance, bulk could be prepared in Europe and exported to the USA, with frozen storage taking place in the USA.

### MODES FOR CARRYING OUT THE INVENTION

Influenza virus A/Wyoming H3N2 is grown in MOCK cell culture in a fermenter by incubating the seed virus with the cells for two days at 35°C. After viral growth, the pH of the fermenter fluid is raised to about 8 by adding of dilute sodium hydroxide, and DNase is added. The culture is maintained at 35°C for four more hours. The culture fluid is then filtered through a depth filter with a nominal pore size of 0.5µm to remove cellular debris.

The resulting suspension of virions is concentrated and purified by ultrafiltration using a membrane with a 300kDa cut-off. Sucrose is added to the concentrate to a final concentration of 30% (w/v) after which formaldehyde is added to a final concentration of 0.015% (w/v). This mixture is stirred at 2-8°C for 72 hours. The virion concentrate is then further purified. Tween 80™ is added to a final concentration of 300µg/ml and cetyltrimethylammonium bromide (CTAB) is added to a final concentration of 750µg/ml. This mixture is stirred at 4°C for three hours, and centrifuged. Collected antigen is incubated overnight at 2-8°C then filtered to remove excess detergent. The HA concentration in the final purified antigen suspension is 400µg/ml. Half of this suspension is frozen at -20°C for future use as a bulk vaccine, and the other half is diluted and formulated as a monovalent vaccine for patient administration.

The frozen bulk vaccine can be thawed and then continue to be used in the same way as the unfrozen bulk vaccine.

It will be understood that the invention has been described by way of example only and modifications may be made whilst remaining within the scope and spirit of the invention.

### REFERENCES (the contents of which are hereby incorporated by reference)

[1] Vaccines. (eds. Plotkin & Orenstein). 4th edition, 2004, ISBN: 0-7216-9688-0.
[2] Tan (2006) Clin Infect Dis 42:S121-4.
[3] WO97/37000.
[4] Brands et al. (1999) Dev Biol Stand 98:93-100.
[5] Halperin et al. (2002) Vaccine 20:1240-7.
[6] Tree et al. (2001) Vaccine 19:3444-50.
[7] Kistner et al. (1998) Vaccine 16:960-8.
[8] Kistner et al. (1999) Dev Biol Stand 98:101-110.
[9] Bruhl et al. (2000) Vaccine 19:1149-58.
[10] Pau et al. (2001) Vaccine 19:2716-21.
[11] *http:*//www.*atcc.org*/
[12] *http:*//*locus.umdnj.edu*/
[13] WO03/076601.
[14] WO2005/042728.
[15] WO03/043415
[16] WO01/85938.
[17] W02006/108846.
[18] EP-A-1260581 (WO01/64846).
[19] WO2006/071563.
[20] WO2005/113758.
[21] WO03/023021
[22] WO03/023025
[23] WO97/37001.
[24] WO2006/027698.
[25] World Health Organisation (2005) Emerging Infectious Diseases 11(10): 1515-21.
[26] Herlocher et al. (2004) J Infecl Dis 190(9):1627-30.
[27] Le et al. (2005) Nature 437(7062):1108.
[28] Hoffmann et al. (2002) Vaccine 20:3165-3170.
[29] Subbarao et al. (2003) Virology 305:192-200.
[30] Liu et al. (2003) Virology 314:580-590.
[31] Ozaki et al. (2004) J. Virol. 78:1851-1857.
[32] Webby et al. (2004) Lancet 363:1099-1103.
[33] WO00/60050.
[34] WO01/04333.
[35] US 6649372.
[36] Neumann et al. (2005) Proc Natl Acad Sci USA 102:16825-9.
[37] W02006/067211.
[38] WO01/83794.
[39] Hoffmann et al. (2000) Virology 267(2):310-7.
[40] WO96/37624.
[41] WO98/46262.
[42] WO95/18861.
[43] WO02/28422.
[44] WO002/067983.
[45] WO02/074336.
[46] WO02/097072.
[47] WO2005/113756.
[48] WO01/21151.
[49] WO01/22992.
[50] Hehme et al. (2004) Virus Res. 103(1-2):163-71.
[51] Treanor et al. (1996) J Infect Dis 173:1467-70.
[52] Keitel et al. (1996) Clin Diagn Lab Immunol 3:507-10.
[53] Lundblad (2001) Biotechnolo and Applied Biochemistry 34:195-197.
[54] Guidance for Industry: Bioanalytical Method Validation. U.S. Department of Health and Human Services Food and Drug Administration Center for Drug Evaluation and Research (CDER) Center for Veterinary Medicine (CVM). May 2001.
[55] Ji et al. (2002) Biotechniques. 32:1162-7.
[56] Briggs (1991) J Parenter Sci Technol. 45:7-12.
[57] Lahijani et al. (1998) Hum Gene Ther. 9:1173-80.
[58] Lokteff et al. (2001) Biologicals. 29:123-32.
[59] EP-B-0870508.
[60] US 5948410.
[61] US patent 6355271.
[62] WO00/23105.
[63] US 5,057,540.
[64] WO96/33739.
[65] EP-A-0109942.
[66] WO96/11711.
[67] WO00/07621.
[68] Barr et al. (1998) Advanced Drug Delivery Reviews 32:247-271.
[69] Sjolanderet et al. (1998) Advanced Drug Delivery Reviews 32:321-338.
[70] Pizza et al. (2000) Int J Med Microbiol 290:455-461.
[71] WO95/17211.
[72] WO98/42375.
[73] Singh et al] (2001) J Cont Release 70:267-276.
[74] WO99/27960.
[75] US 6,090,406
[76] US 5,916,588
[77] EP-A-0626169.
[78] WO99/52549.
[79] WO01/21207.
[80] WO01/21152.
[81] WO02/072012.
[82] Dyakonova et al. (2004) Int Immunopharmacol 4(13):1615-23.
[83] FR-2859633.
[84] Signorelli & Hadden (2003) Int Immunopharmacol 3(8):177-86.
[85] WO2004/064
[86] De Libero et al, Nature Reviews Immunology, 2005, 5: 485-496
[87] US patent 5,936,076.
[88] Oki et al, J. Clin. Investig., 113: 1631-1640
[89] US2005/0192248
[90] Yang et al, Angew. Chem. Int. Ed, 2004, 43: 3818-3822
[91] WO2005/102049
[92] Goff et al, J. Am. Chem., Soc., 2004, 126: 13602-13603
[93] WO03/105769
[94] Cooper (1995) Pharm Biotechnol 6:559-80.
[95] WO03/011223.
[96] Meraldi et al. (2003) Vaccine 21:2485-2491.
[97] Pajak et al. (2003) Vaccine 21:836-842.
[98] US-6586409.
[99] Wong et al. (2003) J Clin Pharmacol 43(7):735-42.
[100] US2005/0215517.
[101] WO90/14837.
[102] Podda & Del Giudice (2003) Expert Rev Vaccines 2:197-203.
[103] Podda (2001) Vaccine 19: 2673-2680.
[104] Vaccine Design: The Subunit and Adjuvant Approach (eds. Powel l & Newman) Plenum Press 1995 (ISBN 0-306-44867-X).
[105] Vaccine Adjuvants: Preparation Methods and Research Protocols (Volume 42 of Methods in Molecular Medicine series). ISBN: 1-59259-083-7. Ed. O'Hagan.
[106] Allison & Byars (1992) Res Immunol 143:519-25.
[107] Hariharan et al. (1995) Cancer Res 55:3486-9.
[108] WO95/11700.
[109] US patent 6,080,725.
[110] WO2005/097181.
[111] WO2006/113373.
[112] Han et al. (2005) Impact of Vitamin E on Immune Function and Infectious Diseases in the Aged at Nutrition, Immune functions and Health EuroConference, Paris, 9-10 June 2005.
[113] US- 6630161.
[114] Hayden et al. (1998) J Clin Invest 101(3):643-9.
[115] Tassignon et al. (2005) Jlmmunol Meth 305:188-98.
[116] Myers et al. (1990) pages 145-156 of Cellular and molecular aspects of endotoxin reactions*.*
[117] Ulrich (2000) Chapter 16 (pages 273-282) of reference 105.
[118] Johnson et al. (1999) J Med Chem 42:4640-9.
[119] Baldrick et al. (2002) Regulatory Toxicol Pharmacol 35:398-413.
[120] US 4,680,338.
[121] US 4,988,815.
[122] WO92/15582.
[123] Stanley (2002) Clin Exp Dermatol 27:571-577.
[124] Wu et al. (2004) Antiviral Res. 64(2):79-83.
[125] Vasilakos et al. (2000) Cell Immunol. 204(1):64-74.
[126] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[127] Jones (2003) Curr Opin Investig Drugs 4:214-218.
[128] WO2004/060308.
[129] W02004/064759.
[130] US 6,924,271.
[131] US2005/0070556.
[132] US 5,658,731.
[133] US patent 5,011,828.
[134] WO2004/87153.
[135] US 6,605,617.
[136] WO02/18383.
[137] W02004/018455.
[138] WO03/082272.
[139] WO2006/002422.
[140] Johnson et al. (1999) Bioorg Med Chem Lett 9:2273-2278.
[141] Evans et al. (2003) Expert Rev Vaccines 2:219-229.
[142] Andrianov et al. (1998) Biomaterials 19:109-115.
[143] Payne et al. (1998) Adv Drug Delivery Review 31:185-196.
[144] Thompson et al. (2003) Methods in Molecular Medicine 94:255-266.
[145] Kandimalla et al. (2003) NucleicAcids Research 31:2393-2400.
[146] WO02/26757.
[147] WO99/62923.
[148] Krieg (2003) Nature Medicine 9:831-835.
[149] McCluskie et al. (2002) FEMS Immunology and Medical Microbiology 32:179-185.
[150] WO98/40100.
[151] US patent 6,207,646.
[152] US patent 6,239,116.
[153] US patent 6,429,199.
[154] Kandimalla et al. (2003) Biochemical Society Transactions 31 (part 3):654-658.
[155] Blackwell et al. (2003) J Immunol 170:4061-4068.
[156] Krieg (2002) Trends Immunol 23:64-65.
[157] WO01/95935.
[158] Kandimalla et al. (2003) BBRC 306:948-953.
[159] Bhagat et al. (2003) BBRC 300:853-861.
[160] WO03/035836.
[161] WO01/22972.
[162] Thompson et al. (2005) J Leukoc Biol 78: 'The low-toxicity versions of LPS, MPL® adjuvant and RC529, are efficient adjuvants for CD4+ T cells'.
[163] UK patent application G-A-2220211.
[164] WO 94/21292.
[165] WO94/00153.
[166] WO95/17210.
[167] WO96/26741.
[168] WO93/19780.
[169] Gennaro (2000) Remington: The Science and Practice of Pharmacy. 20th edition, ISBN: 0683306472.
[170] Banzhoff (2000) Immunology Letters 71 :91-96.
[171] Nony et al. (2001) Vaccine 27:3645-51.
[172] Potter & Oxford (1979) Br Med Bull 35: 69-75.
[173] Greenbaum et al. (2004) Vaccine 22:2566-77.
[174] Zurbriggen et al. (2003) Expert Rev Vaccines 2:295-304.
[175] Piascik (2003) J Am Pharm Assoc (Wash DC). 43:728-30.
[176] Mann et al. (2004) Vaccine 22:2425-9.
[177] Halperin et al. (1979) Am J Public Health 69:1247-50.
[178] Herbert et al. (1979) J Infect Dis 140:234-8.
[179] Chen et al. (2003) Vaccine 21:2830-6.

### EMBODIMENTS

1. A process for preparing an influenza vaccine bulk, comprising the steps of:
   (a) preparing bulk vaccine antigen from a source of influenza virus; and
   (b) storing under frozen conditions the bulk vaccine prepared in step (a).
2. The process of embodiment 1, wherein step (a) is preceded by a step of growing influenza virus, and step (a) involves preparing bulk vaccine antigen from viruses grown in said preceding step.
3. A process for preparing influenza vaccine from such a frozen bulk vaccine, comprising the process of any preceding embodiment, and then the steps of: (c) thawing the frozen bulk vaccine; and (d) preparing a final vaccine for administration to patients from the bulk vaccine.
4. A process for preparing influenza vaccine, comprising the steps of: (a) obtaining a bulk vaccine that has been stored under frozen conditions; and (b) preparing a final vaccine for administration to patients from the bulk vaccine.
5. The process of embodiment 3 or 4, wherein the final vaccine contains a plurality of influenza virus strains.
6. The process of embodiment 5, wherein only one of the plurality was stored as bulk vaccine under frozen conditions.
7. The process of embodiment 5, wherein more than one of the plurality was stored as bulk vaccine under frozen conditions.
8. The process of any preceding embodiment, wherein frozen bulk vaccine is monovalent.
9. The process of any preceding embodiment, wherein bulk vaccine is stored below -10°C.
10. The process of any preceding embodiment, wherein bulk vaccine is stored at about -20°C.
11. The process of any preceding embodiment, wherein the bulk vaccine is stored for ≥2 months.
12. The process of any one of embodiments 3 to 11, wherein the step of preparing a final vaccine involves dilution of the bulk.
13. The process of any one of embodiments 3 to 12, wherein the step of preparing a final vaccine involves addition of a vaccine adjuvant.
14. The process of any one of embodiments 3 to 13, wherein the step of preparing a final vaccine involves mixing the thawed material with antigen prepared from a different influenza virus strain.
15. The process of any one of embodiments 3 to 14, wherein the step of preparing a final vaccine involves filling the vaccine into containers.
16. The process of any preceding embodiment, wherein the influenza virus is grown in eggs.
17. The process of any preceding embodiment, wherein the influenza virus is grown in cell culture.
18. The process of any preceding embodiment, wherein the influenza virus is of HA subtypes H1, H2, H3, H4, H5, H6, H7, H8 or H9.
19. The process of any preceding embodiment, wherein the influenza virus is cold adapted.
20. The process of any preceding embodiment, wherein vaccine is a live virus vaccine.
21. The process of any preceding embodiment, wherein vaccine is an inactivated virus vaccine.
22. The process of embodiment 21, wherein the vaccine is a purified surface antigen vaccine.
23. The process of embodiment 21, wherein the vaccine is a split vaccine.
24. The process of any preceding embodiment, wherein the HA concentration in the frozen bulk is in the range of 75-1000 µg/ml per strain.

## Claims

1. A process for preparing an influenza vaccine bulk, comprising the steps of:
(a) preparing bulk vaccine antigen from a source of influenza virus; and
(b) storing under frozen conditions the bulk vaccine prepared in step (a); wherein the influenza virus is grown in cell culture.

2. The process of claim 1, wherein step (a) is preceded by a step of growing influenza virus, and step (a) involves preparing bulk vaccine antigen from viruses grown in said preceding step.

3. A process for preparing an influenza vaccine from such a frozen bulk vaccine, comprising the process of any preceding claim, and then the steps of:
(c) thawing the frozen bulk vaccine; and
(d) preparing a final vaccine for administration to patients from the bulk vaccine.

4. A process for preparing an influenza vaccine, comprising the steps of:
(a) obtaining a bulk vaccine that has been stored under frozen conditions; and
(b) preparing a final vaccine for administration to patients from the bulk vaccine; wherein the influenza virus is grown in cell culture.

5. The process of claim 3 or 4, wherein the final vaccine contains a plurality of influenza virus strains, optionally:
(a) wherein only one of the plurality was stored as bulk vaccine under frozen conditions; or
(b) wherein more than one of the plurality was stored as bulk vaccine under frozen conditions.

6. The process of any preceding claim, wherein frozen bulk vaccine is monovalent.

7. The process of any preceding claim, wherein bulk vaccine is stored:
(a) below -10°C;
(b) at about -20°C; and/or
(c) for >2 months.

8. The process of any one of claims 3 to 7, wherein the step of preparing a final vaccine involves:
(a) dilution of the bulk;
(b) addition of a vaccine adjuvant;
(c) mixing the thawed material with antigen prepared from a different influenza virus strain; and/or
(d) filling the vaccine into containers.

9. The process of any preceding claim, wherein the influenza virus is of HA subtypes H1, H2, H3, H4, H5, H6, H7, H8 or H9.

10. The process of any preceding claim, wherein the influenza virus is cold adapted.

11. The process of any preceding claim, wherein vaccine is a live virus vaccine.

12. The process of any preceding claim, wherein vaccine is an inactivated virus vaccine, for example, a purified surface antigen vaccine or a split vaccine.

13. The process of any preceding claim, wherein the HA concentration in the frozen bulk is in the range of 75-1000µg/ml per strain.

14. The process of any preceding claim, wherein the influenza virus is grown in a MDCK cell line.

15. The process of claim 14, wherein the MDCK cell line is MDCK 33016.
